# EUROPÄISCHE PATENTSCHRIFT

(11) **EP 0 840 789 B1**
(45) Veröffentlichungstag und Bekanntmachung des Hinweises auf die Patenterteilung: **31.05.2006**
(21) Anmeldenummer: 96928338.1
(22) Anmeldetag: 26.07.1996
(51) Int. Cl.: C12N 15/54, C12N 9/10, C12N 1/21, C07K 16/40, C12Q 1/48, A61K 38/45, C12Q 1/68, G01N 33/573

(54) **TRANSKETOLASE-VERWANDTES PROTEIN**
TRANSKETOLASE-RELATED PROTEIN
PROTEINE APPARENTEE A LA TRANSCETOLASE

(30) Priorität: 27.07.1995 DE 19527552
(43) Veröffentlichungstag der Anmeldung: 13.05.1998
(73) Patentinhaber: Coy, Johannes, Dr., 63762 Grossostheim (DE)
(72) Erfinder: POUSTKA, Annemarie, D-69120 Heidelberg (DE); COY, Johannes, D-63762 Grossostheim (DE)
(74) Vertreter: Rudolph, Ulrike
(86) Internationale Anmeldenummer: PCT/DE1996/001401
(87) Internationale Veröffentlichungsnummer: WO 1997/005253

(56) Entgegenhaltungen:
- EMBL DATABASE, Accession no. U14622, sequence reference HS14622, September 26, 1994; U. HOCHGESCHWENDER: "Rapid identification of gene sequences XP002023967
- EMBL DATABASE, Accession no. Z49258, sequence reference HS14B7, June 5, 1995; K. THOMAS siehe Nukleotiden 36074-36246 XP002023968
- MAMMALIAN GENOME, Bd. 5, 1994, Seiten 131-137, XP000616545 J. COY ET AL: "Identification of tissue-specific expressed sequences in human band Xq28 with complex pig cDNA probes"
- BIOCHEMICAL AND BIOPHYSICAL RESEARCH COMMUNICATIONS, Bd. 183, Nr. 3, 1992, ORLANDO, FL US, Seiten 1159-1166, XP002023964 M. ABEDIDNIA ET AL: "Nucleotide and predicted amino acid sequence of a cDNA clone encoding part of human transketolase "
- JOURNAL OF BIOLOGICAL CHEMISTRY, Bd. 268, Nr. 2, 15.Januar 1993, MD US, Seiten 1397-1404, XP002023965 B. MCCOOL ET AL: "Cloning of human transketolase cDNAs and comparison of the nucleotide sequence of the coding region in Wernicke-Korsakoff and non-Wernicke-Korsakoff individuals"
- DATABASE MEDLINE Accession no. 94106774, Oktober 1993 R. BUTTERWORTH ET AL: "Thiamine-dependent enzyme changes in the brains of alcoholics:....." XP002023969 & ALCOHOLISM, CLINICAL AND EXPERIMENTAL RESEARCH, Bd. 17, Nr. 5, 1993, Seiten 1084-1088,
- DATABASE MEDLINE Accession no. 93187642, Februar 1993 E. JUNG ET AL: "An enzymatic and immunological analysis of transketolase in fibroblasts from Wernicke-Korsakoff syndrome" XP002023970 & JOURNAL OF THE NEUROLOGOCAL SCIENCES, Bd. 114, Nr. 2, 1993, Seiten 123-127,
- GENOMICS 32 (3). 1996. 309-316. ISSN: 0888-7543, 15.März 1996, XP000616489 COY J F ET AL: "Molecular cloning of tissue-specific transcripts of a transketolase-related gene: Implications for the evolution of new vertebrate genes."

## Beschreibung

Die vorliegende Erfindung betrifft ein Transketolase-verwandtes Protein, eine ein solches kodierende DNA und ein Verfahren zur Herstellung eines solchen. Ferner betrifft die Erfindung die Verwendung der DNA und des Proteins sowie gegen das Protein gerichtete Antikörper.

Transketolase ist ein Thiamin-abhängiges Enzym, das den Pentosephosphatzyklus mit der Glykolyse verbindet. Der Pentosephosphatzyklus stellt Zuckerphosphate und NADPH bereit. Transketolase führt überschüssige Zuckerphosphate in die Glykolyse ein, wodurch die Bereitstellung von NADPH unter verschiedenen metabolischen Bedingungen gewährleistet ist. NADPH ist für das Aufrechterhalten von Glutathion im Gehirn wesentlich.

Eine Defizienz von Thiamin ist mit neurologischen Erkrankungen, wie Beriberi und Wernicke-Korsakoff Syndrom, assoziiert. Beriberi äußert sich in akuter Herzinsuffizienz, während sich das Wernicke-Korsakoff Syndrom in akuter Enzephalopathie, gefolgt von chronischer Schädigung des Kurzzeitgedächtnisses zeigt.

Untersuchungen weisen darauf hin, daß eine Thiamin-Defizienz für vorstehende Erkrankungen nicht ursächlich ist. Auch liegt bei Patienten dieser Erkrankungen, z.B. bei Patienten des Wernicke-Korsakoff-Syndroms, keine mutierte Transketolase vor. Die Ursache vorstehender Erkrankungen ist somit bisher nicht bekannt.

Der vorliegenden Erfindung liegt daher die Aufgabe zugrunde, ein Mittel bereitzustellen, mit dem neurologische Erkrankungen, die mit einer Thiamin-Defizienz assoziiert sind, insbesondere Beriberi und Wernicke-Korsakoff Syndrom, in ihrer Ursache untersucht und gegebenenfalls therapiert werden können.

Erfindungsgemäß wird dies durch die Gegenstände in den Patentansprüchen erreicht.

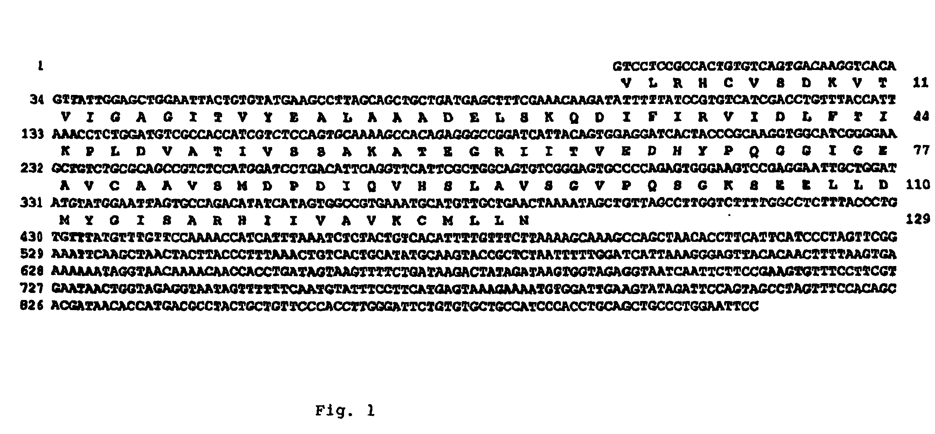

Gegenstand der vorliegenden Erfindung ist somit ein Transketolase-verwandtes Protein, wobei das Protein die Aminosäuresequenz von Fig. 1 umfaßt.

Die vorliegende Erfindung beruht auf der Erkenntnis des Anmelders, daß in Tieren, besonders Säugetieren, ganz besonders dem Menschen, ein Protein existiert, das Homologien zu einer Transketolase, gegebenenenfalls eine Transketolase-Aktivität aufweist, sich aber von einer Transketolase auf dem DNA-Level durch Hybridisierung unter üblichen Bedingungen unterscheidet. Ein solches Protein weist zumindest die Aminosäuresequenz von Fig. 1 auf. Ferner durch eine oder mehrere Aminsoäuren unterschiedliche Aminosäuresequenz liegt das Protein in verschiedenen Geweben, z.B. Gehirn und Herz, in unterschiedlicher Form vor.

In der vorliegenden Erfindung wird vorstehendes Protein mit "Transketolase-verwandtes Protein" (TVP) bezeichnet.

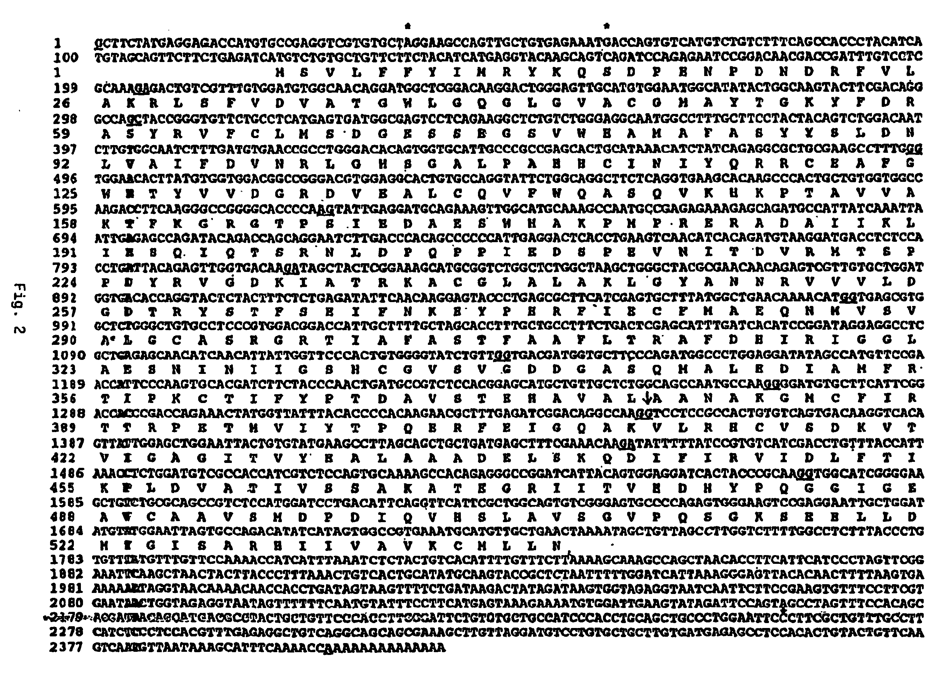

In bevorzugter Ausführungsfrom weist ein (TVP) die Aminosäuresequenz von Fig. 2 auf. Ein solches (TVP) findet sich insbesondere im Gehirn von Tieren, besonders Säugetieren, ganz besonders dem Menschen.

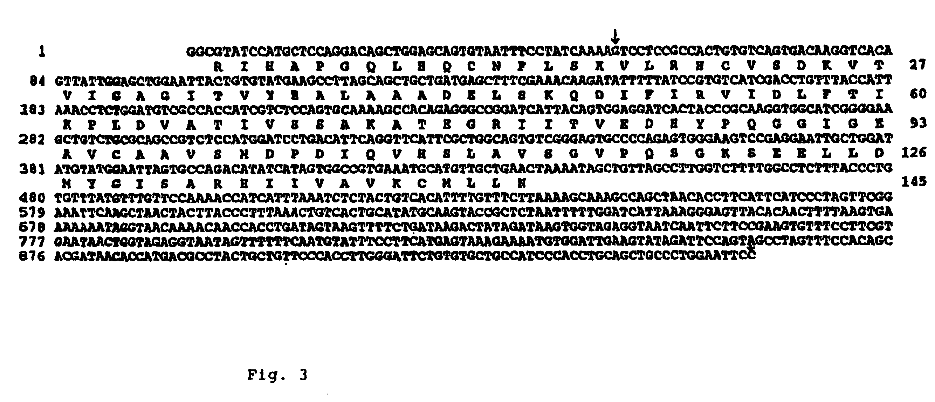

In einer weiteren bevorzugten Ausführungsfrom weist ein (TVP) die Aminosäuresequenz von Fig. 3 auf. Ein solches (TVP) findet sich insbesondere im Herzen von Tieren, besonders Säugetieren, ganz besonders dem Menschen.

Ein weiterer Gegenstand der vorliegenden Erfindung ist eine für ein (TVP) kodierende Nukleinsäure. Dies kann eine RNA oder eine DNA sein. Letztere kann z.B. eine genomische DNA oder eine cDNA sein. Bevorzugt ist eine DNA, die folgendes umfaßt:
die DNA von Fig. 1

Besonders bevorzugt ist die DNA von Fig. 2 und Fig. 3. Die DNA von Fig. 2 kodiert für ein (TVP), das insbesondere im Gehirn von Tieren, besonders Säugetieren, ganz besonders dem Menschen, vorliegt. Die DNA von Fig. 3 kodiert für ein (TVP), das insbesondere im Herzen von Tieren, besonders Säugetieren, ganz besonders dem Menschen, vorliegt. Die DNA von Fig. 2 wurde bei der DSM (Deutsche Sammlung von Mikroorganismen und Zellkulturen) als JFC 317 unter DSM 9994 am 23. Mai 1995 hinterlegt.

Nachstehend wird eine erfindungsgemäße DNA in Form einer cDNA beschrieben. Diese steht beispielhaft für jede unter die vorliegende Erfindung fallende DNA.

Zur Herstellung einer erfindungsgemäßen cDNA ist es günstig, von einer Cosmid-Bibliothek, z. B. q1Z (vgl. Dietrich, A. et al., Nucleic Acids Res. 19, (1991), 2567-2572) auszugehen, von der Klone die Region Xq28 des menschlichen Genoms umfassen. Solche Klone werden auf einer Filtermembran fixiert und mit markierten, aus mRNA von Schweinegeweben, z.B. Gehirn, Muskel, Leber, Herz, durch reverse Transkription erhaltenen cDNA-Pools hybridisiert (vgl. Coy, J.F. et al., Mammalian Genome 5, (1994) 131-137). Diejenigen Klone, die mit den cDNA-Pools ein Hybridisierungssignal aufweisen, werden zum Screening einer menschlichen cDNA-Bibliothek, z.B. von fötalem Gehirn-, Herzgewebe, verwendet. Hierfür eignet sich besonders die cDNA-Bibliothek A-Zap, Stratagene, Kat.-Nr. 936206. Es wird eine erfindungsgemäße cDNA erhalten.

Eine erfindunggemäße cDNA kann in einem Vektor bzw. Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8, wobei letzterer bevorzugt ist. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A.

Der Fachmann kennt geeignete Zellen, um eine, erfindungsgemäße, in einem Expressionsvektor vorliegende cDNA zu exprimieren. Beispiele solcher Zellen umfassen die E.coli-Stämme HB101, DH1, x1776, JM101, JM 109, BL21 und SG 13009, wobei letzterer bevorzugt ist, den Hefe-Stamm Saccharomyces cerevisiae und die tierischen Zellen L, 3T3, FM3A, CHO, COS, Vero und HeLa sowie die Insektenzellen sf9.

Der Fachmann weiß, in welcher Weise eine erfindungsgemäße cDNA in einen Expressionsvektor inseriert werden muß. Ihm ist auch bekannt, daß diese DNA in Verbindung mit einer für ein anderes Protein bzw. Peptid kodierenden DNA inseriert werden kann, so daß die erfindungsgemäße cDNA in Form eines Fusionsproteins exprimiert werden kann.

Des weiteren kennt der Fachmann Bedingungen, transformierte bzw. transfizierte Zellen zu kultivieren. Auch sind ihm Verfahren bekannt, das durch die erfindungsgemäße cDNA exprimierte Protein zu isolieren und zu reinigen. Ein solches Protein, das auch ein Fusionsprotein sein kann, ist somit ebenfalls Gegenstand der vorliegenden Erfindung.

Ein weiterer Gegenstand der vorliegenden Erfindung ist ein gegen ein vorstehendes Protein bzw. Fusionsprotein gerichteter Antikörper. Ein solcher Antikörper kann durch übliche Verfahren hergestellt werden. Er kann polyklonal bzw. monoklonal sein. Zu seiner Herstellung ist es günstig, Tiere, insbesondere Kaninchen oder Hühner für einen polyklonalen und Mäuse für einen monoklonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

Die vorliegende Erfindung ermöglicht es, neurologische Erkrankungen, die mit einer Thiamin-Defizienz assoziiert sind, wie Beriberi und Wernicke-Korsakoff Syndrom, in ihrer Ursache zu untersuchen. Mit einem erfindungsgemäßen Antikörper kann (TVP) in Körperflüssigkeiten von Personen nachgewiesen werden. Es kann eine Beziehung von (TVP) zur Entstehung und Ausbildung vorstehender Erkrankungen hergestellt werden. Ferner kann mit einem erfindungsgemäßen (TVP) ein gegen dieses Protein gerichteter Autoantikörper nachgewiesen werden. Beide Nachweise können durch übliche Verfahren, insbesondere einen Western Blot, einen ELISA, eine Immunpräzipitation oder durch Immunfluoreszenz, erfolgen. Desweiteren kann mit einer erfindungsgemäßen Nukleinsäure, insbesondere einer DNA und hiervon abgeleiteten Primern, die Expression des für (TVP) kodierenden Gens nachgewiesen werden. Dieser Nachweis kann in üblicher Weise, insbesondere in einem Southern Blot, erfolgen.

Darüberhinaus eignet sich die vorliegende Erfindung, Maßnahmen für und gegen das Vorliegen von (TVP) in Personen zu ergreifen. Mit einem erfindungsgemäßen Antikörper kann (TVP) an Personen inhibiert werden. Andererseits kann mit einem erfindungsgemäßen (TVP), insbesondere nach Kopplung an ein vom Körper nicht als fremd angesehenes Protein, z.B. Transferrin oder BSA, die Menge von (TVP) in Personen erhöht werden. Entsprechendes kann auch mit einer erfindungsgemäßen Nukleinsäure, insbesondere einer DNA, erreicht werden, die unter die Kontrolle eines in bestimmten Geweben, z.B. Gehirn, Herz, induzierbaren Promotors gestellt wird und nach ihrer Expression zur Bereitstellung von (TVP) in diesen Geweben führt. Darüberhinaus kann eine erfindungsgemäße Nukleinsäure, insbesondere eine DNA, auch zur Inhibierung von (TVP) genutzt werden. Hierzu wird die Nukleinsäure, z.B. als Basis für die Erstellung von Anti-Sinn-Oligonukleotiden zur Expressions-Inhibierung des für (TVP) kodierenden Gens verwendet.

Die vorliegende Erfindung stellt somit einen großen Beitrag zur diagnostischen und therapeutischen Erfassung von neurologischen Erkrankungen, die mit einer Thiamin-Defizienz assoziiert sind, insbesondere Beriberi und Wernicke-Korsakoff Syndrom, dar.

### Kurze Beschreibung der Zeichnungen:

- Fig. 1: zeigt die Basensequenz und die davon abgeleitete Aminosäuresequenz eines erfindungsgemäßen (TVP),
- Fig. 2: zeigt die Basensequenz und die davon abgeleitete Aminosäuresequenz eines erfindungsgemäßen Gehirn-spezifischen (TVP) (Pfeile geben die Lage von (TVP) von Fig. 1 an), und
- Fig. 3: zeigt die Basensequenz und die davon abgeleitete Aminosäuresequenz eines erfindungsgemäßen Herz-spezifischen (TVP) (Pfeile geben die Lage von (TVP) von Fig. 1 an).

Die vorliegende Erfindung wird durch die nachstehenden Beispiele erläutert.

### Beispiel 1: Herstellung und Reinigung eines erfindungsgemäßen (TVP)

Zur Herstellung eines erfindungsgemäßen (TVP) wurde die DNA von Fig. 2 bzw. 3 als Template verwendet. Es wurde ein PCR-Verfahren durchgeführt. Bezüglich der DNA von Fig. 2 wurde als Primer-Paar verwendet: 5'-CAGAGATCTATGAG-GTACAAGCAGTCAG-3' und 5'-GGGAAGCTTTTAGTTCAGCAACATGC-3'. Bezüglich der DNA von Fig. 3 wurde als Primer-Paar verwendet: 5'-CAGAGATCTATGTGGCGTATCCATGC-3' und 5'GGGAAGCTTTTAGTTCAGCAACATGC-3'. Der PCR-Ansatz bzw. die PCR-Bedingungen waren jeweils wie folgt:

| **PCR-Ansatz** | |
|---|---|
| Template DNA (Fig. 1) | : 1µl = 1 ng |
| Pfu-Polymerase 10x-Puffer | : 10*µ*l = 1 x |
| DMSO | : 10µl = 10 % |
| dNTP's | : 1*µ*L = je 200µM |
| Oligonukleotide, je 1,5*µ*l | : 3*µ*l = je 150 ng |
| H₂O-bidest | : ad 99µl |

### PCR-Bedingungen

- 92°C - 5 min
- Zugabe von 1µl Pfu-Polymerase (Stratagene) = 2,5 Einheiten
- Zugabe von Paraffin

| **PCR** | |
|---|---|
| 92°C 1 min | |
| 58°C 1 min | 1 Zyklus |
| 72°C 10 min | |
| 92°C 1 min | |
| 58°C 1 min | 39 Zyklen |
| 72°C 2 min | |
| 72°C 10min | 1 Zyklus |

Die amplifizierte DNA wurde jeweils mit Bgl II und Hind III gespalten und in den mit Bgl II- und Hind III gespaltenen Expressionsvektors pQE-8 (Diagen) inseriert. Es wurde das Expressionsplasmid pQ/TVP-G (pQ/TVP-H) erhalten. Ein solches kodiert für ein Fusionsprotein aus 6 Histidin-Resten (N-Terminuspartner) und dem erfindungsgemäßen (TVP) von Fig. 2 (Fig.3) (C-Terminuspartner). pQ/TVP-G (pQ/TVP-H) wurde zur Transformation von E.co(i SG 13009(vgl. Gottesman, S. et al., J. Bacteriol. 148, (1981), 265-273) verwendet. Die Bakterien wurden in einem LB-Medium mit 100 µg/ml Ampicillin und 25Eµg/ml Kanamycin kultiviert und 4 h mit 60µM Isopropyl-β-D-Thiogalactopyranosid (IPTG) induziert. Durch Zugabe von 6 M Guanidinhydrochlorid wurde eine Lyse der Bakterien erreicht, anschließend wurde mit dem Lysat eine Chromatographie (Ni-NTA-Resin) in Gegenwart von 8 M Harnstoff entsprechend der Angaben des Herstellers (Diagen) des Chromatographie-Materials durchgeführt. Das gebundene Fusionsprotein wurde in einem Puffer mit pH 3,5 eluiert. Nach seiner Neutralisierung wurde das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigte sich, daß ein erfindungsgemäßes (Fusions)protein in hochreiner Form hergestellt werden kann.

### Beispiel 2: Herstellung und Nachweis eines erfindungsgemäßen Antikörpers

Ein erfindungsgemäßes Fusionsprotein von Beispiel 1 wurde einer 18 % SDS-Polyacrylamid-Gelelektrophorese unterzogen. Nach Anfärbung des Gels mit 4 M Natriumacetat wurde eine ca. 59 (16,5) kD Bande aus dem Gel herausgeschnitten und in Phosphat gepufferter Kochsalzlösung inkubiert. Gel-Stücke wurden sedimentiert, bevor die Proteinkonzentration des Überstandes durch eine SDS-Polyacrylamid-Gelelektrophorese, der eine Coomassie-Blau-Färbung folgte, bestimmt wurde. Mit dem Gel-gereinigten Fusionsprotein wurden Tiere wie folgt immunisiert:

### Immunisierungsprotokoll für polyklonale Antikörper im Kaninchen

Pro Immunisierung wurden 35µg Gel-gereinigtes Fusionsprotein in 0,7 ml PBS und 0,7 ml komplettem bzw. inkomplettem Freund's Adjuvans eingesetzt.

| | | |
|---|---|---|
| Tag O: | 1. | Immunisierung (komplettes Freund's Adjuvans) |
| Tag 14: | 2. | Immunisierung (inkomplettes Freund's Adjuvans; icFA) |
| Tag 28: | 3. | Immunisierung (icFA) |
| Tag 56: | 4. | Immunisierung (icFA) |
| Tag 80: | | Ausbluten |

Das Serum des Kaninchens wurde im Immunoblot getestet. Hierzu wurde ein erfindungsgemäßes Fusionsprotein von Beispiel 1 einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wurde das Nitrocellulosefilter eine Stunde bei 37°C mit einem ersten Antikörper inkubiert. Dieser Antikörper war das Serum des Kaninchens (1:10000 in PBS). Nach mehreren Waschschritten mit PBS wurde das Nitrocellulosefilter mit einem zweiten Antikörper inkubiert. Dieser Antikörper war ein mit alkalischer Phosphatase gekoppelter monoklonaler Ziege Anti-Kaninchen-IgG-Antikörper (Dianova) (1:5000) in PBS. Nach 30-minütiger Inkubation bei 37°C folgten mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar waren.

Es zeigte sich, daß erfindungsgemäße, polyklonale Antikörper hergestellt werden können.

### Immunisierungsprotokoll für polyklonale Antikörper im Huhn

Pro Immunisierung wurden 40µg Gel-gereinigtes Fusionsprotein in 0,8 ml PBS und 0,8 ml komplettem bzw. inkomplettem Freund's Adjuvans eingesetzt.

| | |
|---|---|
| Tag O. | 1. Immunisierung (komplettes Freund's Adjuvans) |
| Tag 28: | 2. Immunisierung (inkomplettes Freund's Adjuvans; icFA) |
| Tag 50: | 3. Immunisierung (icFA) |

Aus Eigelb wurden Antikörper extrahiert und im Western Blot getestet. Es wurden erfindungsgemäße, polyklonale Antikörper nachgewiesen.

### Immunisierungsprotokoll für monoklonale Antikörper der Maus

Pro Immunisierung wurden 12*µ*g Gel-gereinigtes Fusionsprotein in 0,25 ml PBS und 0,25 ml komplettem bzw. inkomplettem Freund's Adjuvans eingesetzt; bei der 4. Immunisierung war das Fusionsprotein in 0,5 ml (ohne Adjuvans) gelöst.

| | | |
|---|---|---|
| Tag O. | 1. | Immunisierung (komplettes Freund's Adjuvans) |
| Tag 28: | 2. | Immunisierung (inkomplettes Freund's Adjuvans; icFA) |
| Tag 56: | 3. | Immunisierung (icFA) |
| Tag 84: | 4. | Immunisierung (PBS) |
| Tag 87: | | Fusion |

Überstände von Hybridomen wurden im Western Blot getestet. Erfindungsgemäße, monoklonale Antikörper wurden nachgewiesen.

## Patentansprüche

1. Transketolase-verwandtes Protein, wobei das Protein die Aminosäuresequenz von Fig. 1 umfaßt.

2. Transketolase-verwandtes Protein **dadurch gekennzeichnet, daß** es die Aminosäuresequenz von Fig. 2 umfaßt.

3. Transketolase-verwandtes Protein **dadurch gekennzeichnet, daß** es die Aminosäuresequenz von Fig. 3 umfaßt.

4. Nukleinsäure, **dadurch gekennzeichnet, daß** sie die Basensequenz von Fig. 1 umfaßt.

5. Nukleinsäure, **dadurch gekennzeichnet, daß** sie die Basensequenz von Fig. 2 umfaßt.

6. Nukleinsäure , **dadurch gekennzeichnet, daß** sie die Basensequenz von Fig. 3 umfaßt.

7. Expressionsplasmid, **dadurch gekennzeichnet, daß** es eine Nukleinsäure nach einem der Ansprüche 4 bis 6 umfaßt.

8. Transformante **dadurch gekennzeichnet, daß** sie das Expressionsplasmid nach Anspruch 7 enthält.

9. Verfahren zur Herstellung eines Proteins nach einem der Ansprüche 1 bis 3, **dadurch gekennzeichnet, daß** es die Kultivierung der Transformante nach Anspruch 8 umfaßt.

10. Antikörper, **dadurch gekennzeichnet, daß** er spezifisch gegen ein Protein nach einem der Ansprüche 1 bis 3 gerichtet ist.

11. Verwendung eines Proteins nach einem der Ansprüche 1 bis 3 zur Herstellung eines Mittels für die Diagnose oder Therapie von neurologischen Erkrankungen, die mit einer Thiamin-Deffizienz assoziiert sind, wie beispielsweise Beriberi und Wernicke-Korsakoff-Syndrom.

12. Verwendung einer Nukleinsäure nach einem der Ansprüche 4 bis 6 zur Herstellung eines Mittels für die Diagnose oder Therapie von neurologischen Erkrankungen, die mit einer Thiamin-Deffizienz assoziiert sind, wie beispielsweise Beriberi und Wernicke-Korsakoff-Syndrom.

13. Verwendung eines Antikörpers nach Anspruch 10 zur Herstellung eines Mittels für die Diagnose oder Therapie von neurologischen Erkrankungen, die mit einer Thiamin-Deffizienz assoziiert sind, wie beispielsweise Beriberi und Wernicke-Korsakoff-Syndrom.

## Claims

1. Transketolase-related protein comprising the amino acid sequence of fig. 1.

2. Transketolase-related protein comprising the amino acid sequence of fig. 2.

3. Transketolase-related protein comprising the amino acid sequence of fig. 3.

4. Nucleic acid comprising the base sequence of fig. 1.

5. Nucleic acid comprising the base sequence of fig. 2.

6. Nucleic acid comprising the base sequence of fig. 3.

7. Expression plasmid comprising a nucleic acid of any one of claims 4 to 6.

8. A transformant comprising the Expression plasmid of claim 7.

9. Method for producing a protein according to any one of claims 1 to 3, wherein said method comprises cultivation of the transformant of claim 8.

10. Antibody **characterised in that** it is specifically directed against a protein of any one of claims 1 to 3.

11. Use of a protein according to any one of claims 1 to 3 for producing an agent for diagnosis of therapy of neurologic diseases associated with a thiamine deficiency as for example, beriberi and Wernicke-Korsakoff syndrome.

12. Use of a nucleic acid according to any one of claims 4 to 6 for producing an agent for diagnosis of therapy of neurologic diseases associated with a thiamine deficiency as for example , beriberi and Wernicke-Korsakoff syndrome.

13. Use of an antibody according to claim 1 o for producing an agent for diagnosis of therapy of neurologic diseases associated with a thiamine deficiency as for example , beriberi and Wernicke-Korsakoff syndrome.

## Revendications

1. Protéine apparentée à la transcétolase, la protéine comprenant la séquence d'acides aminés de la figure 1.

2. Protéine apparentée à la transcétolase, **caractérisée en ce qu'**elle comprend la séquence d'acides aminés de la figure 2.

3. Protéine apparentée à la transcétolase, **caractérisée en ce qu'**elle comprend la séquence d'acides aminés de la figure 3.

4. Acide nucléique, **caractérisé en ce qu'**il comprend la séquence de base de la figure 1.

5. Acide aminé, **caractérisé en ce qu'**il comprend la séquence de bases de la figure 2.

6. Acide aminé, **caractérisé en ce qu'**il comprend la séquence de bases de la figure 3.

7. Plasmide d'expression, **caractérisé en ce qu'**il comprend un acide nucléique selon l'une des revendications 4 à 6.

8. Transformant, **caractérisé en ce qu'**il comprend le plasmide d'expression selon la revendication 7.

9. Procédé pour la fabrication d'une protéine selon l'une des revendications 1 à 3, **caractérisé en ce qu'**il comprend la culture du transformant selon la revendication 8.

10. Anticorps, **caractérisé en ce qu'**il est dirigé spécifiquement contre une protéine selon la revendication 1 à 3.

11. Utilisation d'une protéine selon l'une des revendications 1 à 3 pour la fabrication d'un moyen pour le diagnostic ou la thérapie de maladies neurologiques qui sont associées à une déficience de la thiamine, telles que par exemple le béribéri et le syndrome de Wemicke-Korsakoff.

12. Utilisation d'un acide nucléique selon l'une des revendications 4 à 6 pour la fabrication d'un moyen pour le diagnostic ou la thérapie de maladies neurologiques qui sont associées à une déficience de la thiamine, comme par exemple le béribéri et le syndrome de Wemicke-Korsakoff.

13. Utilisation d'un anti-corps selon la revendication 10 pour la fabrication d'un moyen pour le diagnostic ou la thérapie de maladies neurologiques qui sont associées à une déficience de la thiamine, comme par exemple de béribéri et le syndrome de Wemicke-Korsakoff.
